Europäisches Patentamt

European Patent Office

Office européen des brevets

(19)

(11) Numéro de publication: **0 214 885**
**B1**

(12) **FASCICULE DE BREVET EUROPEEN**

(45) Date de publication du fascicule du brevet: 26.09.90

(51) Int. Cl.⁵: **A 61 F 2/34**

(21) Numéro de dépôt: **86401700.9**

(22) Date de dépôt: **30.07.86**

(54) Composant cotyloidien de prothèse de hanche, à implanter sans ciment.

(30) Priorité: **12.08.85 FR 8512277**

(43) Date de publication de la demande:
**18.03.87 Bulletin 87/12**

(45) Mention de la délivrance du brevet:
**26.09.90 Bulletin 90/39**

(84) Etats contractants désignés:
**AT BE CH DE GB IT LI LU NL SE**

(56) Documents cités:
**WO-A-86/02261**
**DE-A-3 119 130**
**FR-A-2 095 854**
**FR-A-2 233 976**
**FR-A-2 242 065**
**FR-A-2 377 798**
**FR-A-2 416 004**
**FR-A-2 548 012**
**FR-A-2 554 342**
**US-A-3 584 318**

(73) Titulaire: **Epinette, Jean-Alain**
**27 rue Lamandin**
**F-62700 Bruay-en-Artois (FR)**
(73) Titulaire: **Carlier, Yves**
**13 rue Pierre Lhermitte**
**F-80000 Amiens (FR)**
(73) Titulaire: **Duthoit, Etienne**
**8 allée des Hêtres Louvil**
**F-59830 Cysoing (FR)**

(72) Inventeur: **Epinette, Jean-Alain**
**27 rue Lamandin**
**F-62700 Bruay-en-Artois (FR)**
Inventeur: **Carlier, Yves**
**13 rue Pierre Lhermitte**
**F-80000 Amiens (FR)**
Inventeur: **Duthoit, Etienne**
**8 allée des Hêtres Louvil**
**F-59830 Cysoing (FR)**

(74) Mandataire: **CABINET BONNET-THIRION**
**95 Boulevard Beaumarchais**
**F-75003 Paris (FR)**

Courier Press, Leamington Spa, England.

## Description

L'invention a trait à des prothèses de hanche, et plus particulièrement à un composant cotyloïdien de prothèse de hanche, destiné à être implanté sans ciment et comprenant une calotte métallique à fixer dans la cavité cotyloïde convenablement préparée, avec un axe polaire orienté sensiblement dans la direction moyenne du col du fémur, et une cupule en matériau polymère adaptée à s'emboîter exactement dans la calotte pour former garniture de frottement et accueillir la tête sphérique d'un composant fémoral en sorte de reproduire l'articulation naturelle.

A l'origine les composants de prothèse, fémoraux et cotyloïdiens, étaient fixés respectivement dans le fémur et dans le cotyle de l'os iliaque à l'aide d'un ciment polymérisable, généralement de type acrylique. Cette technique présente quelques inconvénients, avec notamment la création, dans les os intéressés, d'efforts dirigés dans des directions non naturelles, source de douleurs, et la perte de la plasticité au vieillissement, avec création de jeu entre l'os et le composant correspondant.

On a créé des composants de prothèse de hanche aptes à être implantés sans ciment, en assurant un ancrage mécanique solide du composant dans l'os qui le reçoit. Dans le cas des composants cotyloïdiens, qui sont envisagés ici il existe deux types de structure.

Dans un premier type, le composant comporte une calotte métallique en anneau, généralement tronconique, munie d'un filetage extérieur qui doit se mettre en prise avec un filetage complémentaire pratiqué dans la périphérie de la paroi du cotyle. Ces anneaux sont fréquemment autotaraudants, avec des filetages interrompus à arêtes tranchantes. La mise en place de la prothèse en précontrainte doit assurer l'irréversibilité du vissage. Toutefois, il peut se produire à la longue une ostéolyse du cotyle, avec prise de jeu du composant. En outre le passage des lignes de force depuis le fémur jusqu'à l'os iliaque est quelque peu différent de ce qu'il est dans l'articulation naturelle saine, avec une obliquité par rapport aux directions de plus grande résistance naturelle de la charpente osseuse.

Le document FR—A—2,554,342 décrit une prothèse de ce premier type; cependant la calotte est de forme extérieure exactement sphérique, et présente, avec un orifice polaire éventuel, un secteur sphérique partant du bord équatorial muni d'ailettes interrompues qui forment filetage autotaraudant. La calotte sphérique au-delà du secteur sphérique, est lisse et vient porter exactement sur le toit du cotyle. La forme hémisphérique de la calotte a été choisie pour que la préparation de la cavité n'exige que l'enlèvement superficiel d'os dur. Pour obtenir un ancrage sûr et sans jeu, le filetage est à pas progressif. L'ancrage peut être consolidé par du ciment.

Dans un second type, le composant comprend une calotte approximativement hémisphérique, dont la surface extérieure est garnie de métal poreux. Ce type de composant, créé à l'origine pour améliorer la liaison entre le composant et le ciment, s'est montré apte à favoriser une liaison directe entre le composant métallique et l'os spongieux en croissance, qui s'infiltre dans les pores du métal. Toutefois ces composants ne sont ancrés dans l'os par ce mécanisme de croissance, dit "réhabitation" qu'après un laps de temps qui se compte en semaines. Aussi il est prévu un ancrage primaire, obtenu par des vis traversant des passages dans la calotte et engagés dans des trous taraudés pratiqués dans la paroi du cotyle.

On notera que ces vis, qui matérialisent le contact primaire entre la calotte du composant et le cotyle définissent un passage préférentiel pour les lignes de force. Par une disposition appropriée des vis, on peut restituer sensiblement le trajet des lignes de force de l'articulation naturelle saine en correspondance avec les directions de plus grande résistance de la charpente osseuse. Ceci est important pour réduire les séquelles douloureuses de la prothèse, et faciliter la rééducation du sujet.

Toutefois l'ancrage primaire par vis est relativement fragile, surtout lorsque le sujet présente un vieillissement des os.

Dans le but d'assurer un ancrage primaire résistant et sûr, et un ancrage secondaire excellent par réhabitation, l'invention propose un composant cotyloïdien de prothèse de hanche, destinée à être implantée sans ciment et comprenant une calotte métallique à fixer dans la cavité cotyloïde convenablement préparée, avec un axe polaire orienté sensiblement dans la direction moyenne du col du fémur, et une cupule en matériau polymère adaptée à s'emboîter exactement dans la calotte pour former garniture de frottement et accueillir la tête sphérique d'un composant fémoral, en sorte de reproduire l'articulation naturelle, la calotte, de forme approchant un hémisphère, comportant un orifice polaire et, coaxial à l'axe polaire et occupant un premier secteur sphérique de fixation partant du bord équatorial, un filetage interrompu à arêtes vives propre à s'engager dans la paroi de la cavité cotyloïde en creusant son passage, caractérisé en ce qu'un second secteur sphérique de fixation accolé au premier secteur et s'étendant jusqu'à l'orifice polaire comporte des plages garnies de métal poreux apte à être envahi par de l'os spongieux en croissance depuis la paroi de cavité cotyloïde.

Grâce à ces dispositions, la prothèse bénéficie d'un ancrage primaire solide par les éléments de filetage autotaraudants, ce qui autorise une mise en charge précoce de l'articulation restaurée. Dans une seconde phase, la croissance d'os spongieux à partir du toit du cotyle, qui produit une interpénétration de l'os et des plages de métal poreux, vient interdire tout jeu du filetage. On notera que les efforts principaux résultant de la mise en charge de l'articulation se traduisent essentiellement par une mise en compression des zones de liaison entre les plages de métal poreux et le toit du cotyle, et que la résistance au dévissage du filetage se produit par des efforts de cisaillement des zones de liaison sensiblement dans leur plan, où la résistance de la liaison est maximale.

De préférence le composant cotyloïdien comporte, de part et d'autre d'une ligne séparant les deux secteurs sphériques, des trous aptes à laisser passer des vis pénétrant dans la paroi de cavité cotyloïde, pour bénéficier des avantages liés à la pose de telles vis, canalisation des lignes de forces dans les directions naturelles, et consolidation de l'ancrage primaire. Les trous sont de préférence régulièrement espacés angulairement autour de l'axe polaire.

De préférence le filetage interrompu comporte deux filets imbriqués, ce qui permet d'augmenter le pas et en corollaire réduire le nombre de tours lors de la mise en place, et équilibrer les efforts à l'engagement du filetage de part et d'autre de l'axe. En disposition préférée, le filetage interrompu est supprimé à l'intérieur de fuseaux sphériques régulièrement espacés angulairement, et des plages garnies de métal poreux sont disposées là où le filetage est supprimé. Cette disposition est prévue pour améliorer, après réhabitation osseuse, la résistance au dévissage de la prothèse.

De préférence le bord équatorial de la calotte comporte des créneaux pour recevoir des tétons correspondants d'outils de vissage, et le bord de cupule présente des tétons qui s'engagent dans les créneaux. Avantageusement les créneaux et les tétons présentent des formes complémentaires pour un engagement à baïonnette, afin d'améliorer le maintien de la cupule dans la calotte.

En disposition préférée la cupule présente une cavité interne qui épouse la sphère du composant fémoral sur un peu plus qu'une hémisphère, la lèvre terminale de la cupule permettant par élasticité le passage de la sphère du composant fémoral, pour retenir cette sphère par la suite.

En disposition préférée les plages de métal poreux sont constituées de particules de métal frittées, et la calotte est en titane, ainsi que les plages de métal poreux, en raison de la légéreté du titane, de sa résistance mécanique élevée, et de son excellente compatibilité avec les tissus et notamment le tissu osseux.

Les caractéristiques et avantages de l'invention ressortiront d'ailleurs de la description qui va suivre à titre d'exemple, en référence aux dessins annexés dans lesquels:

la figure 1 est une vue en élévation, à axe vertical, d'une calotte de composant cotyloïdien selon l'invention;

la figure 2 est une vue en coupe d'un composant cotyloïdien;

la figure 3 est une vue par en dessous du composant selon la figure 2;

la figure 4 est une vue éclatée d'une prothèse de hanche incorporant un composant cotyloïdien;

la figure 5 est une vue de détail de l'assemblage d'une cupule dans une calotte d'un composant cotyloïdien.

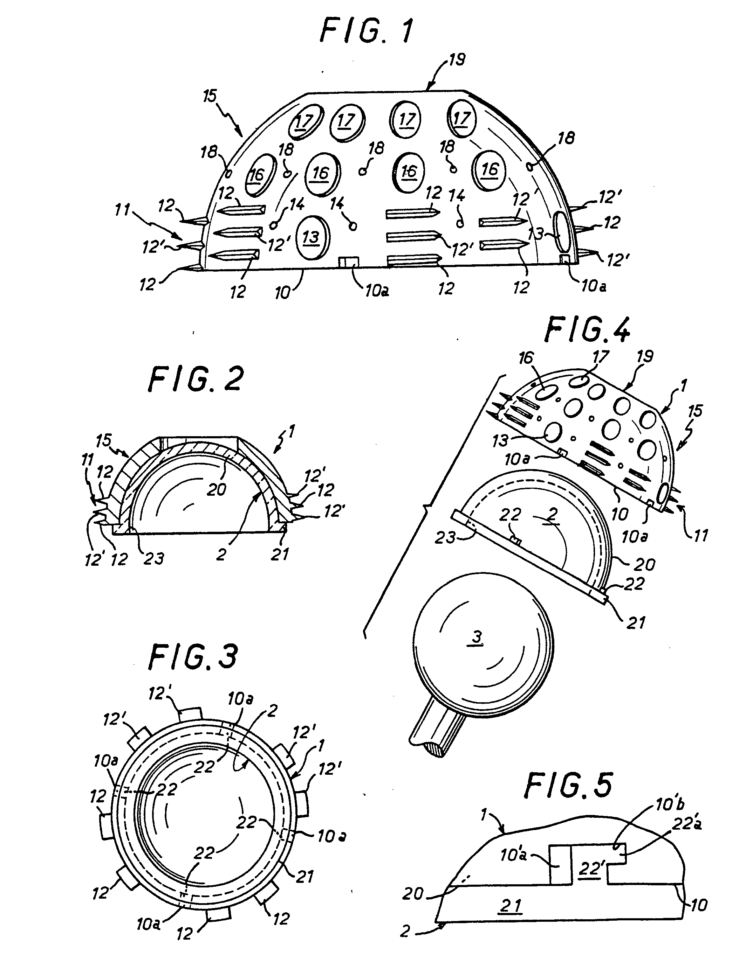

Selon le mode de réalisation choisi et représenté aux figures 1 à 3, le composant cotyloïdien comprend une calotte extérieure 1, métallique, et une cupule 2 en matériau polymère, qui s'emboîte exactement dans la calotte 1 pour former garniture de frottement.

La calotte 1, réalisée de préférence en titane, présente, une forme générale hémisphérique, avec un orifice polaire 19, et deux secteurs sphériques 11 et 15, étagés, le premier secteur 11 partant du bord équatorial 10, tandis que le second secteur 15 se termine à la périphérie de l'orifice polaire 19. Ces deux secteurs sont dévolus à la fixation dans la cavité, cotyloïde convenablement préparée.

Le secteur 11 comporte un filetage interrompu 12, 12' à double filet. Les interruptions du filetage 12, 12' forment deux séries 12 et 12' de dents à arêtes tranchantes, qui forment taraud de façon connue. Les interruptions sont en alignement méridien, de sorte que les dents se situent dans une série de fuseaux sphériques. En outre les dents sont supprimées dans un fuseau sur trois.

Dans le secteur sphérique 15, entre le premier secteur 11 et l'orifice polaire 19, sont disposées des pastilles 16, 17 formées de particules de titane frittées. Ces pastilles sont, selon la représentation de la figure 1, disposées dans les fuseaux sphériques définis par les interruptions du filetage 12, 12', en deux rangées 16 et 17 parallèles au bord équatorial 10.

En outre des pastilles semblables 13 sont disposées dans le premier secteur sphérique 11, là où les dents sont supprimées.

De part et d'autre d'une ligne parallèle au bord équatorial 10, qui sépare les secteurs sphériques 11 et 15, sont ménagés des trous, 14 dans le secteur 11 et 18 dans le secteur 15, propres à laisser passer des vis de fixation. Ces trous 14 et 18 sont régulièrement espacés angulairement autour de l'axe polaire de la calotte 1.

Dans le bord équatorial 10 sont ménagés 4 créneaux 10a, disposés en croix, où peuvent prendre des tétons d'outils de vissage, de façon connue.

On signale que l'orifice polaire 19 est destiné à permettre au chirurgien de vérifier la mise en place de la calotte 1 dans la cavité cotyloïde, et éventuellement d'insérer des greffons osseux pour accélérer l'ancrage secondaire du composant.

Dans la calotte 1, comme on le voit mieux à la figure 2, une cupule 2 en matériau polymère, ici polyéthylène, vient s'insérer exactement à l'intérieur de la calotte 1. La structure de cette cupule 2 est représentée plus clairement aux figures 3 et 4. La cupule 2 se compose d'une partie hémisphérique 20 d'épaisseur régulière, avec un rebord 21 situé en dessous du plan équatorial de la cupule 2, et tangent à ce plan. Des tétons 22 sont ménagés pour pénétrer dans les créneaux 10a de la calotte 1, et maintenir la cupule 2 immobile en rotation par rapport à la calotte 1.

Comme on le comprendra en référence à la figure 4, la cupule en polyéthylène 2 vient s'insérer dans la calotte 1 après que celle-ci ait été fixée dans la cavité cotyloïde convenablement préparée, la fixation comprenant le vissage autotaraudant du filetage 12, 12', et éventuellement la mise en place de vis à travers des trous choisis 14 ou 18. L'insertion de la cupule 2 comprend la mise en place des tétons 22 dans les créneaux 10a. Après insertion de la cupule 2, la tête sphérique 3 d'un composant fémoral est introduite dans la cupule, en forçant pour écarter la lèvre 23 du rebord 21. Le retour élastique de la lèvre 23 assure une certaine retenue de la tête sphérique 3 à l'intérieur de la cupule 2, sans pour autant gêner les pivotements de la tête 3. On remarquera que la tension des muscles, et, en position debout du sujet, la charge de la prothèse sous l'effet du poids du corps sont normalement suffisants pour éviter le déboîtement de la tête 3. Toutefois, lors de la pose de la prothèse, il est avantageux que la tête sphérique soit en légère retenue dans la cupule 2, notamment pour vérifier que la tête 3 est bien en place, et qu'aucun corps étranger ne fait obstacle à cette mise en place correcte.

Par ailleurs on aura noté que l'orientation des composants 1, 2 et 3, avec l'axe principal incliné à 30° sur la grande dimension de la feuille, rappelle l'orientation des composants de prothèse par rapport à la verticale du sujet debout.

La figure 5 représente une variante de réalisation des éléments complémentaires créneaux 10'a du bord équatorial 10, téton 22' de la cupule 2. Dans cette disposition le créneau 10'a comporte une échancrure 10'b parallèle au bord 10 à distance de celui-ci, et le téton 22' possède un prolongement 22'a propre à s'engager dans l'échancrure 10'b en fixation à baïonnette. On remarquera que la largeur du téton 22' avec son prolongement 22'a correspond à celle du créneau 10'a sans son échancrure 10'b, pour permettre, bien entendu, l'engagement.

Cette disposition permet un maintien sûr de la cupule 2 dans la calotte 1. Comme il a été remarqué à propos du maintien de la tête sphérique 3 dans la cupule 2, l'intérêt de la disposition réside essentiellement dans la vérification de la mise en place de la cupule.

On aura remarqué que la description ne s'étend pas sur la pratique opératoire de la pose de la prothèse de hanche, cette pratique étant en dehors du cadre de l'invention. Par ailleurs le composant cotyloïdien est conçu pour permettre une pose facile et efficace, et des suites opératoires favorables, tout en laissant au chirurgien de larges possibilités d'adaptation aux cas particuliers.

Dans cet esprit, l'invention n'est pas limitée aux exemples décrits, mais en embrasse tous les modes d'exécution dans le cadre des revendications.

## Revendications

1. Composant cotyloïdien de prothèse de hanche, destinée à être implantée sans ciment et comprenant une calotte métallique (1) à fixer dans la cavité cotyloïde convenablement préparée, avec un axe polaire orienté sensiblement dans la direction moyenne du col du fémur, et une cupule (2) en matériau polymère adaptée à s'emboîter exactement dans la calotte (1) pour former garniture de frottement et accueillir la tête sphérique (3) d'un composant fémoral, en sorte de reproduire l'articulation naturelle, la calotte (1), de forme approchant un hémisphère, comportant un orifice polaire (19) et coaxial à l'axe polaire et occupant un premier secteur sphérique (11) de fixation partant du bord équatorial, un filetage (12, 12') interrompu à arêtes vives propre à s'engager dans la paroi de la cavité cotyloïde en creusant son passage, caractérisé en ce qu'un second secteur sphérique (15) de fixation accolé au premier secteur (11) et s'étendant jusqu'à l'orifice polaire (19) comporte des plages (16, 17) garnies de métal poreux apte à être envahi par de l'os spongieux en croissance depuis la paroi de cavité cotyloïde.

2. Composant cotyloïdien selon la revendication 1, caractérisé en ce qu'il comporte, de part et d'autre d'une ligne séparant les deux secteurs sphériques, des trous (14, 18) aptes à laisser passer des vis pénétrant dans la paroi de cavité cotyloïde.

3. Composant cotyloïdien selon la revendication 2, caractérisé en ce que les trous (14, 18) de passage de vis sont régulièrement espacés angulairement autour de l'axe polaire.

4. Composant cotyloïdien selon une quelconque des revendications 1 à 3, caractérisé en ce que ledit filetage interrompu (12, 12') comporte deux filets imbriqués (12 et 12').

5. Composant cotyloïdien selon une quelconque des revendications 1 à 4, caractérisé en ce que ledit filetage interrompu (12, 12') est en outre supprimé à l'intérieur de fuseaux sphériques régulièrement espacés angulairement autour de l'axe polaire, des plages (13) garnies de métal poreux étant disposées là où le filetage est supprimé.

6. Composant cotyloïdien selon une quelconque des revendications 1 à 5, caractérisé en ce que ledit bord équatorial (10) présente des créneaux (10a, 10'a) adaptés à recevoir des tétons d'outils de vissage, le bord de cupule présentant des tétons (22, 22') aptes à s'engager dans ces créneaux.

7. composant cotyloïdien selon la revendication 6, caractérisé en ce que les créneaux (10'a) de bord équatorial (10) de calotte et les tétons (22') de cupule correspondants présentent des formes complémentaires (10'b, 22'a) aptes à un engagement réciproque à baïonnette.

8. Composant cotyloïdien selon une quelconque des revendications 1 à 7, caractérisé en ce que ladite cupule (2) présente une cavité interne adaptée à épouser la tête sphérique du composant fémoral (3) au-delà d'une hémisphère, avec une lèvre (23) terminale déformable élastiquement, en sorte de laisser pénétrer ladite tête sphérique (3) dans la cavité interne et de l'y retenir.

9. Composant cotyloïdien selon une quelconque des revendications 1 à 8, caractérisé en ce que les plages de métal poreux (16, 17, 14) sont constituées de particules de métal frittées.

10. Composant cotyloïdien selon une quelconque des revendications 1 à 9, caractérisé en ce que la calotte (1) est en titane.

**Patentansprüche**

1. Hüftgelenkprothesenpfanne, die ohne Zement implantierbar ist und folgende Teile aufweist: eine Kalotte (1) aus Metall zur Befestigung in der geeignet vorbereiteten Hüftgelenkpfannenhöhlung mit einer polaren Achse, die im wesentlichen in der mittleren Richtung des Femurhalses ausgerichtet ist, und einen Becher (2) aus Polymermaterial, der genau in die Kalotte (1) passend ausgebildet ist, um eine Gleitschalung zu bilden und den Kugelkopf (3) eines Femurelements aufzunehmen, so daß die natürliche Artikulation reproduziert wird, wobei die Kalotte (1) von angenäherter Halbkugelform eine polare Öffnung (19) und koaxial zur polaren Achse und in einem vom Äquatorrand sich erstreckenden, der Befestigung dienenden ersten Kugelabschnitt (11) ein unterbrochenes Gewinde (12, 12') mit scharfen Kanten aufweist, das geeignet ist, sich selbstschneidend in die Wand der Hüftgelenkpfannenhöhlung einzuschrauben, dadurch gekennzeichnet, daß ein der Befestigung dienender zweiter Kugelabschnitt (15) an den ersten Kugelabschnitt (11) anschließt, sich bis zur polaren Öffnung (19) erstreckt und Bereiche (16, 17) aufweist, die mit porösem Metall versehen sind, in das von der Wand der Hüftgelenkpfannenhöhlung her spongiöser Knochen hineinwachsen kann.

2. Hüftgelenkprothesenpfanne nach Anspruch 1, dadurch gekennzeichnet, daß sie beiderseits einer die zwei Kugelabschnitte trennenden Linie Löcher (14, 18) aufweist, durch welche in die Wand der Hüftgelenkpfannenhöhlung eindringende Schrauben hindurchtreten können.

3. Hüftgelenkprothesenpfanne nach Anspruch 2, dadurch gekennzeichnet, daß die Löcher (14, 18) für den Durchtritt von Schrauben in regelmäßigen Winkelabständen um die polare Achse verteilt sind.

4. Hüftgelenkprothesenpfanne nach einem der Ansprüche 1 bis 3, dadurch gekennzeichnet, daß das unterbrochene Gewinde (12, 12') zwei dachziegelartig angeordnete Gewinde (12 und 12') aufweist.

5. Hüftgelenkprothesenpfanne nach einem der Ansprüche 1 bis 4, dadurch gekennzeichnet, daß das unterbrochene Gewinde (12, 12') außerdem im Inneren von in regelmäßigen Winkelabständen um die polare Achse angeordneten Kugelzweiecken weggelassen ist, wobei mit porösem Metall versehene Bereiche (13) dort angeordnet sind, wo das Gewinde weggelassen ist.

6. Hüftgelenkprothesenpfanne nach einem der Ansprüche 1 bis 5, dadurch gekennzeichnet, daß der Äquatorrand (10) Kerben (10a, 10'a) zur Aufnahme von Zapfen eines Schraubwerkzeugs aufweist und der Rand des Bechers Zapfen (22, 22') aufweist, die in diese Kerben eingreifen können.

7. Hüftgelenkprothesenpfanne nach Anspruch 6, dadurch gekennzeichnet, daß die Kerben (10'a) am Äquatorrand (10) der Kalotte und die entprechenden Zapfen (22') des Bechers komplementäre Formen (10'b, 22'a) aufweisen, die bajonettartig ineinander eingreifen-können.

8. Hüftgelenkprothesenpfanne nach einem der Ansprüche 1 bis 7, dadurch gekennzeichnet, daß der Becher (2) eine Innenhöhlung aufweist, welche den Kugelkopf des Femurelements (3) jenseits einer Halbkugel aufnehmen kann und eine elastisch verformbare Randlippe (23) aufweist, welche den Kugelkopf (3) in die Innenhöhlung eindringen läßt und dort zurückhält.

9. Hüftgelenkprothesenpfanne nach einem der Ansprüche 1 bis 8, dadurch gekennzeichnet, daß die Bereiche aus porösem Metall (16, 17, 13) aus Teilchen aus Sintermetall bestehen.

10. Hüftgelenkprothesenpfanne nach einem der Ansprüche 1 bis 9, dadurch gekennzeichnet, daß die Kalotte (1) aus Titan besteht.

**Claims**

1. A cotyloid component of a hip prosthesis intended to be implanted without cement and comprising a metal dome (1) to be fixed in a suitably prepared cotyloid cavity, having a polar axis directed substantially in the medial direction of the femur neck, and a cup (2) of polymer material to be fitted precisely into the dome (1) so as to form a friction lining and to accommodate the spherical head (3) of a femoral component in such a way as to reproduce a natural joint, the dome (1), which is of approximately hemispherical shape, comprising a polar opening (19) and, coaxial to the polar axis and occupying a first spherical fastening sector (11) starting from the equatorial edge, a discontinuous screw thread (12, 12') with sharp crests able to engage in the wall of the cotyloid cavity while cutting a passage therein, characterised in that a second spherical fastening sector (15) adjoining the first sector (11) and extending up to the polar opening (19) comprises areas (16, 17) provided with porous metal capable of being invaded by spongy bone growing from the wall of the cotyloid cavity.

2. A cotyloid component according to claim 1, characterised in that, on either side of a line separating the two spherical sectors, it comprises holes (14, 18) through which screws can pass to penetrate the wall of the cotyloid cavity.

3. A cotyloid component according to claim 2, characterised in that the holes (14, 18) for the passage of screws are angularly regularly spaced about the polar axis.

4. A cotyloid component according to any one of claims 1 to 3, characterised in that said discontinuous screw thread (12, 12') comprises two overlapping threads (12 and 12').

5. A cotyloid component according to any one of claims 1 to 4, characterised in that said discontinuous screw thread (12, 12') is additionally omitted within spherical zones regularly spaced angularly about the polar axis, areas (13) provided with porous metal being disposed at the locations where the thread is omitted.

6. A cotyloid component according to any one of claims 1 to 5, characterised in that said equatorial edge (10) has slots (10a, 10'a) adapted to receive the projections of screwing implements, the edge of the cup being provided with projections (22, 22') adapted to engage in said slots.

7. A cotyloid component according to claim 6, characterised in that the slots (10'a) of the equatorial edge (10) of the dome and the corresponding projections (22') of the cup are of complementary shape (10'b, 22'a) suitable for bayonet-type mutual engagement.

8. A cotyloid component according to any one of claims 1 to 7, characterised in that said cup (2) has an inner cavity which conforms to the spherical head of the femoral component (3) to a greater extent than a hemisphere, with an end lip (23) which is elastically deformable in such a way as to allow said spherical head (3) to penetrate into the inner cavity and to retain it therein.

9. A cotyloid component according to any one of claims 1 to 8, characterised in that the areas of porous metal (16, 17, 14) are composed of sintered metal particles.

10. A cotyloid component according to any one of claims 1 to 9, characterised in that the dome (1) is made of titanium.

## FIG. 1

## FIG. 2

## FIG. 3

## FIG.4

## FIG.5